# EUROPEAN PATENT APPLICATION

(11) **EP 2 106 746 A1**
(43) Date of publication of application: **07.10.2009**
(21) Application number: 08153956.1
(22) Date of filing: 02.04.2008
(51) Int. Cl.: A61B 5/00, G01N 27/30

(54) **Monitoring target endogenous species**

(71) Applicant: National University of Ireland, Maynooth, Maynooth, County Kildare (IE)
(72) Inventor: Lowry, John, 5 Dublin (IE); Finnerty, Niall, Bray, Wicklow (IE); Brown, Finbar, 15 Dublin (IE)
(74) Representative: Lane, Cathal Michael

(57) **Abstract**

An electrode comprising a conducting substrate for detecting species such as nitric oxide (NO), carbon monoxide (CO), oxygen (O₂) and hydrogen (H₂) and a polymer matrix formed from a first layer only or first and second layers with the second layer applied to the first. The matrix forms a permselective barrier. Each layer has a first pre-coat or first and second pre-coats. Each pre-coat may be formed by: depositing a liquid form of at least one halogenated polymer such as fluorinated or chlorinated polymers onto a substrate and allowing the material to dry. A liquid form of at least one halogenated polymer adheres the first and second layers to the substrate. The electrode allows for detection of the target species and allows real time *in vivo* measurements to be taken. Blood flow can also be monitored. An electrode bundle with electrodes for different target species is also provided.

## Description

### Field of the Invention

The invention is a device, such as a biosensor for example a micro-electrochemical sensor, than can selectively monitor species in the body. The present invention relates to detecting species in body fluids. Such species include gaseous species within the body such as NO (nitric oxide), CO (carbon monoxide), O₂ (oxygen) and H₂. The species may be naturally occurring within the body such as NO, CO or O₂. Determining the naturally occurring amounts may be the objective. Alternatively a species may be introduced and monitored. A given species may be detected indirectly for example by conversion to an electroactive species. The species may be present in any part of a human or animal body, for example in the brain. Desirably the sensor will be suitable for sustained real-time monitoring.

Detection of any species can be subject to interference by (other) interfering species. Accordingly the invention is directed toward selectively detecting the target species. More particularly it can be difficult to detect a number of species at any given time because of the need to selectively detect each without interference from the others.

### Background and Brief Description of Related Art

The device of the present invention will generally be an electrode-based sensor ("EBS"). An EBS is suitable for use as a biosensor. It may be configured to detect a species which is in sufficiently close proximity to the electrode to be detected by the electrode. Such EBSs are known.

Generally the following characteristics apply to sensors. (1) The response time of the electrode has to be sufficiently rapid to follow the changes in concentration due to reaction or for example clearance of the species from the body. (2) The detection limit has to be low enough to detect the concentrations present. (3) The electrode requires high selectivity, in order to obtain a genuine species signal without interference from other electroactive species. (4) For certain applications, such as in monitoring a specific brain region, for example to monitor local changes, the electrode must be small in order to obtain spatially resolved detection. (5) The electrode should have high stability so it can be used more than once. (6) The electrode should be easy to fabricate and handle. (7) The electrode must have a robust nature, for example an ability to function under relatively hostile and non-controllable conditions for example the conditions within the body such as within the living brain. (8) The electrode should have sufficient biocompatibility.

Electrode bundles of various types are known. The present inventors are interested in providing an electrode bundle that is suitable for use in monitoring different species simultaneously.

### Nitric Oxide

Nitric oxide (NO) is a gaseous, paramagnetic radical and is one of the smallest molecules found in nature. It is thought that NO, or a related compound, is an endothelium-derived relaxing factor. It may also be involved in thrombosis; inflammation; immunity; and neurotransmission. NO is a gas, which means it can diffuse freely through cell membranes, and as a result has many functions both intra/extracellularly. It regulates vascular tone, acts as a neuronal signal in the gastrointestinal tract and central nervous system, and is assumed to contribute to the pathology of several diseases including Hypertension, Schizophrenia, Parkinson's and Alzheimer's disease.

Like all free radicals, NO is extremely reactive and has a high affinity for interaction with ferrous hemoproteins such as soluble guanylate cyclase and hemoglobin while also reacting readily with oxygen, peroxides and the superoxide anion (O₂ ⁻). The difficulty in measuring NO in biological specimens is further exasperated due to the small concentrations (nanomolar or lower levels) that exist in biological samples. The presence of a large number of possible interfering species present at relatively high concentrations in biological systems, such as ascorbic acid (AA), uric acid (UA), the catecholamines and their metabolites, and the products of NO oxidation such as nitrite (NO₂) and nitrate (NO₃), makes the requirement for an NO analysis technique to be both specific and sensitive.

The majority of NO monitoring methods that are currently available measure NO indirectly and are hindered by significant drawbacks. The most commonly used methods for NO detection found in the literature are electron paramagnetic resonance (EPR), chemiluminescence, UV-visible spectroscopy, electrochemistry, mass spectroscopy and gas chromatography. Use of each of these technologies are complicated, for some the detection limit is too high (not sensitive enough) and in general none allow for real-time monitoring.

For direct measurement electrodes have been employed. For example, electrodes have been modified by transition metal complexes, gas permeable membranes and heme (also haem) proteins, to impart NO selectivity.

For successful *in vivo* NO monitoring, therefore: (1) The response time of the electrode has to be extremely fast so as to follow the changes in NO concentration as it reacts with other species. (2) The detection limit has to be low as NO is present at extremely small concentrations in the body such as in the ECF of the brain. (3) The electrode requires high selectivity, in order to obtain a genuine NO signal without interference from other electroactive species. Oxidation is the preferred method of NO sensor detection due to an interference problem encountered with O₂ when the sensor is operating in the reduction mode.

The most commonly used selective membranes are applied by casting or by electropolymerisation. Materials used include nitrocellulose, cellulose acetate, polymer combinations, α-cyclodextrin and Nafion®. The thickness of cast membranes is more difficult to control and therefore reproducibility is sometimes a problem. In contrast, electropolymerised films are more reproducible and can also be manufactured more thinly providing a more rapid response. Both conducting and non-conducting electropolymerised films have been used with the latter been utilized on a more regular basis.

Shibuki designed the first NO sensor that was believed to indicate the possibility for in vivo NO monitoring (c.f. Shibuki, K. (1990) An electrochemical microprobe for detecting nitric oxide release in brain-tissue. Neuroscience Research, 9, 69-76.). The electrode is based on capillary tube containing a Pt electrode in acidic solutions with a gas-permeable membrane to provide selectivity. It is thought that such sensors are best suited for monitoring low NO concentrations over short periods of time (c.f. Christodoulou, D., Kudo, S., Cook, J. A., Krishna, M. C., Miles, A., Grisham, M. B., Murugesan, R., Ford, P. C., and Wink, D. A. (1996) Electrochemical methods for detection of nitric oxide. Nitric Oxide, Pt A - Sources and Detection of No; No Synthase, 268, 69-83.)

Malinski and Taha (c.f. Malinski, T. and Taha, Z. (1992) Nitric oxide release from a single cell measured insitu by a porphyrinic-based microsensor. Nature, 358, 676-678.) lowered the oxidation potential of NO to improve the performance of NO sensors. Their carbon fibre sensor had nickel porphyrin which was electropolymerised onto the surface of a carbon fibre electrode that was then subsequently followed by a single Nafion® coat. Although the characteristics obtained by Malinski and Taha's electrode are excellent, it has been reported that this electrode is not only hard to replicate, but is also prone to interference from dopamine (DA) (c.f. Archer, S. (1993) Measurement of nitric oxide in biological models. Journal of the Federation of American Societies for Experimental Biology, 7, 349-360).

Carbon fibre electrodes coated with Ni-THMPP and Nafion® have also been used for NO detection.

Commercial electrodes for NO can be purchased from World Precision Instruments Inc ("WPI" based in Stevenage, Hertfordshire SG2 7EG England), which include those sold under the trade names ISO-NOPF, ISO-NOPMC, ISO-NOP, ISO-NOP30L, ISO-NOP30 and the ISO-NOP007. The surface of the sensor is modified by multilayered NO-selective membrane. The electrodes have a response time of <5 s and an LOD (limit of detection) of 0.2 nM. It is thought that these sensors have temperature sensitivity issues. According to Paterson, D. (2003) Professor of Cardiovascular Physiology, University of Oxford: Personal Communication electrodes of this type may not perform satisfactorily in biological samples.

Friedemann et al. (c.f. Friedemann, M. N., Robinson, S. W., and Gerhardt, G. A. (1996) o-phenylenediamine modified carbon fiber electrodes for the detection of nitric oxide. Analytical Chemistry, 68, 2621-2628) designed a carbon fibre electrode utilizing two selective membranes. The carbon fibre was first modified with thermally annealed Nafion® (3 coats of 5% Nafion® annealed at 200°C for 5 minutes). After the Nafion® membrane had been successfully applied, a further selective membrane was electropolymerised onto the electrodes active surface. An insulating form of *o*-PD (*o-*PD is (*o*)-phenylenediamine ) was plated on the carbon surface (to form a polymer coating of polyphenylenediame). Sensitivity was good and a rapid response with an average response time of ca. 514 ms was achieved.

A design protocol for the Nitric Oxide (NO) sensor is a Nafion® Pre-coat Application technique. Nafion® is a polymer which acts a selective membrane by forming a negatively charged layer rejecting anions (negatively charged species) from the electrode surface. Use of Nafion® is described by Brown and Lowry (Brown, F.O. and Lowry, J.P. (2003) Microelectrochemical Sensors for In Vivo Brain Analysis; An Investigation of Procedures for Modifying Pt electrodes using Nafion®. Analyst, 128, 700-705). The Nafion® pre-coat method is as follows: a droplet of Nafion® is placed onto a watch glass and allowed to air dry at room temperature - this is known as a pre-coat. After the Nafion® drop has dried (due to evaporation of alcohol) further drops are placed on top (5 pre-coats) until a concentrated layer of Nafion® remains. A final drop of Nafion® is then placed onto the pre-coats, which allows the concentrated layer to adhere to the active surface of the sensor following dipping (application). The sensor is allowed to air dry for 2 minutes before being placed in an oven at 210 °C for 5 minutes. The desired protocol is 5 pre-coats, 2 applications. The aforementioned sensor design has maximised the parameters required for an NO sensor, that is, its sensitivity towards NO, its selectivity against the other species present in the brain, e.g. dopamine, ascorbic acid, *etc*. However, this sensor has a relatively slow response time of the order of 30 s - a fast response time is very important, as NO is a very short-lived species.

There is still a requirement for a sensor which is full characterisable *in-vivo* and which can thus give accurate real-time measurements of NO concentrations. In particular it is desirable to provide a sensor which is accurate but has faster response times.

### Carbon Monoxide

It is also generally desirable to monitor CO (carbon monoxide) levels in bodily fluids. Carbon monoxide is naturally occurring in the body and may form carboxy haemoglobin. Carbon monoxide may be produced as a breakdown of haem. Endogenously produced CO is thought to have important physiological roles in the body similar to those of NO such as neurotransmitter or a blood vessels relaxant. In addition CO regulates inflammatory reactions. Levels of carbon monoxide in the body can be a health indicator with higher levels indicating a potential health issue. Relatively high levels of carbon monoxide in the body can indicate some form of poisoning which may be manifested as hypoxia. Symptoms of poisoning can look similar to those of other conditions including, Parkinson's disease, vertigo etc. It is also an important factor in determining the ability of blood to carry oxygen. CO occurs at higher levels in smokers. It is thus desirable to monitor levels of CO in the body.

### Hydrogen

H₂ is suitable as a blood flow tracer as it is metabolically inert and normally present in the body tissues. H₂ may be administered by inhalation and monitored by amperometric detection at an implanted electrode. Furthermore, H₂ dissolves readily in lipids and defuses rapidly in tissues, thus it penetrates nervous tissues as well. Finally because of its low water:gas partition coefficient of 0.018, the pulmonary circulation should rapidly remove it from arterial blood. The rate of the decay of the current produced by H₂ is proportional to the rate of blood flow. So for example a blood low such as a regional Cerebral Blood Flow (rCBF) may be measured. Lowry & Finn (c.f.: Lowry, J.P. and Finn, T. (2003) A Microelectrochemical Sensor for Real-Time Monitoring of Regional Cerebral Blood Flow. Monitoring Molecules in Neuroscience, Stockholm, Sweden) describe utilising two bare Pt electrodes, one for *in situ* generation of H₂ the other for its detection. An electrode bundle comprising a generation and recording electrode was thus created. The decay curve of the H₂ detected was recorded when H₂ generation was ceased. Absence of cross-talk (interference between electrodes) was also reported for the electrodes in question.

### Oxygen

Molecular oxygen was one of the first substances detected voltammetrically *in-vivo,* both in brain and in peripheral tissue. Carbon paste electrodes (CPEs) have been previously used to monitor and measure brain tissue oxygen levels in awake, freely-moving animals. The major advantage that CPEs have in neurochemical studies is their stability over extended periods of repeated recording.

However, noble metal electrodes (platinum/iridium (Pt/Ir)) have significant advantages over CPEs; for example, the smaller size of the sensor (typically 25-125 µm in diameter) reduces the damage that results from implantation in the brain (CPEs are typically 300 µm in diameter). However, a disadvantage of metal-based oxygen electrodes is that they are susceptible to surface poisoning, unlike carbon paste oxygen electrodes, and therefore require the use of protecting membranes.

Notwithstanding the various teachings of the prior art, there is still a necessity to provide a reliable and accurate EBS which can accurately sense the target species, reject interfering species, and operate in in-vivo conditions. The ability to provide simultaneous data on different species is also desirable.

### Summary of the Invention

The present invention provides an electrode comprising:
(i) a conducting substrate for detecting a target species selected from CO, H₂ and O₂;
(ii) a polymer matrix which forms a permselective barrier for the conducting substrate;
   the polymer matrix comprising a layer formed by:
   (a) depositing a liquid form of at least one halogenated polymer such as fluorinated or chlorinated polymers for example, perfluoro, perchloro and perfluorochloro polymers, onto a substrate,
   (b) allowing the material to dry,
   (c) depositing a liquid form of at least one halogenated polymer such as fluorinated or chlorinated polymers for example, perfluoro, perchloro and perfluorochloro polymers, onto the dried material,
   (d) optionally repeating steps (b) and (c);
   depositing a liquid form of at least one halogenated polymer such as fluorinated or chlorinated polymers for example, perfluoro, perchloro and perfluorochloro polymers, onto the layer thus formed; and
   utilising the material deposited on the layer to adhere the layer to the conducting substrate so as to form a permselective barrier for the conducting substrate. The electrodes of the invention are suitable for detecting gaseous molecules. The polymer matrix is permeable to gaseous molecules.

The present invention also provides an electrode comprising:
(i) a conducting substrate;
(ii) a polymer matrix which forms a permselective barrier for the conducting substrate and formed from:
   a. a first layer, or
   b. first and second layers wherein the second layer is applied to the first, the polymer matrix forming a permselective barrier for the conducting substrate;
   the, or each layer comprising a first pre-coat or first and second pre-coats said first pre-coat being formed by:
   (a) depositing a liquid form of at least one halogenated polymer such as fluorinated or chlorinated polymers onto a substrate, and
   (b) allowing the material to dry; and
      said first and second pre-coats being formed by:
   (c) depositing a liquid form of at least one halogenated polymer such as fluorinated or chlorinated polymers onto a substrate,
   (d) allowing the material to dry;
   (e) depositing a liquid form of at least one halogenated polymer such as fluorinated or chlorinated polymers, onto the dried material, and
   (f) allowing the material to dry;
   said first layer being adhered to the conducting substrate and said second layer being adhered to the first layer by depositing a liquid form of at least one halogenated polymer such as fluorinated or chlorinated polymers on the layer; and utilising the material deposited on the layer to adhere the layer to the conducting substrate or the first layer so as to form the permselective barrier for the conducting substrate. The electrodes of the invention are suitable for detecting gaseous molecules. The polymer matrix is permeable to gaseous molecules.

An electrode according to the present invention is capable of providing a signal directly proportional to the concentration of the species being detected and are particularly useful for detecting gaseous species. Electrode according to the invention have proven to be very sensitive. In each aspect of the invention the electrode will be configured for detecting the target species. In particular the electrode will be configured at an appropriate potential. For example, the electrode may be at an oxidative or reductive potential as is appropriate.

Such an electrode can work as a sensor and has shown permselectivity against interferents *in vivo.* The response time sufficiently short making electrodes of the invention particularly suitable for *in-vivo* measuring. The electrode signal is reliable and reproducible. This is achieved without any substantial interference from such potential interfering species. In particular the present invention allows changes of target species concentrations to be monitored in real time. One aspect of the present invention allows the monitoring of two or more species at one time utilising two or more electrodes.

An electrode of the present invention configured for detection of NO has proven to be very sensitive towards NO. It can detect physiological NO concentration (of up to 1µM) or above. Generally the limit of detection is calculated as 3x Standard Deviation of the blank/M, where M represents the sensitivity of the electrode. For a Type 1 sensor (see below) the limit of detection was 234mM which is very significant. Electrodes prepared according to either aspect of the present invention and configured for detection of O₂ provide protection against surface poisoning yet do not show any significant difference in O₂ sensitivity compared with bare platinum. Similar results are possible for H₂ and CO also.

The H₂ sensor of the invention can be utilised to monitor blood-flow.

It has been possible to characterise sensors of the invention. For example a Nitric Oxide sensor including an electrode of the invention has been characterised fully. Also further sensor characterisation has indicated that the NO sensor was not affected by physiological concentrations of O₂. Furthermore it was not affected by increased temperatures, for example a temperature of 37°C. A response time of about 14 seconds was displayed at physiological temperature. Real-time *in-vivo* neurochemical monitoring of NO has huge potential in determining and finding possible cures for a number of neuro-degenerative and psychiatric disorders. NO determination in various body fluids, such as extracellular fluid, for example brain fluid is important in many applications. This compares well to other electrodes.

It will be appreciated that in the context of the present invention the expression "dried" or "allowing the material to dry" is intended to indicate that the material is dried before the next application. It does not preclude the possibility of actively drying the material. Allowing the material to air dry is however preferred. Drying may employ heat if desired.

Generally the invention will involve allowing the material to dry sufficiently under ambient conditions so as to at least partially form a solid material. Typically the time involved is low for example from about 1 to about 5 minutes such as from about 1 to about 4 minutes such as about 2 minutes. The substrate onto which the liquid form is deposited is not the conducting substrate. It can be any other suitable substrate.

Suitably the liquid form of at least one halogenated polymer is a solution thereof. Drying therefore comprises removal of solvent. As a liquid form the polymer may be applied drop-wise to build up the layer as required. Suitable solvents include: alcohols such as lower aliphatic alcohols.

It will be thus appreciated that each layer or pre-coat may be formed by droplet evaporation. As discussed above droplet evaporation can occur under ambient conditions. This is a simple and reliable method of construction.

As a further example the polymer matrix may be formed by first and second layers each layer comprising a first pre-coat. In another construction the polymer matrix is formed by a first layer and said layer comprises first and second pre-coats.

In one embodiment the polymer matrix formed is dip-coated once utilising a liquid form of at least one halogenated polymer such as fluorinated or chlorinated polymers. In another embodiment the polymer matrix is dip-coated twice utilising a liquid form of at least one halogenated polymer such as fluorinated or chlorinated polymers. Desirably the dip-coating material is a less-concentrated liquid form such as a 20% or less, for example a 15% or less, such as a 10% or less for example about a 5% solution of the (liquid form of the) material being deposited to form the matrix.

For the first aspect of the invention above it is desirable that each layer is built up by depositing the material and allowing it to dry at least three times and no greater than seven times, for example the layer may be built up by depositing the material and allowing it to dry at least four times and no greater than six times such as five times.

Generally, (including dip-coating steps,) it is desirable that there is no greater than four applications of the halogenated polymer in total. This achieves good permselectivity and excellent response times as set out above. For example two applications (two pre-coats) to form the layer (and later dip-coating twice may be employed). Also, each of two layers (one applied to the other), each layer comprising two pre-coats is a desirable construction..

It is desirable that there are no greater than three applications of the halogenated polymer in total. For example one pre-coat forming a layer which is applied and then dip-coating twice. Alternatively two pre-coats form a layer which is then dip-coated once.

Each application builds up the material by depositing the material and allowing it to dry. This stepwise deposition of material forms the layer with a permselective character.

Suitable drying times (at the temperatures above) are from about 1 to about 10 minutes, such as from about 2 to about 9 minutes, such as about 3 to about 8 minutes, for example from about 4 to about 7 minutes, such as about 5 minutes.

It is generally desirable to anneal the polymer matrix formed or any layer(s) within the matrix. For example the polymer matrix may have only a first layer and that layer is annealed. In an alternative arrangement the polymer matrix comprises first and second layers and the first layer is annealed. In a further arrangement according to the present invention the polymer matrix comprises first and second layers and the first layer and second layers are each annealed. Annealing results in a cured matrix that has different characteristics than a dried matrix. Generally annealing of a first layer occurs before application of the second. In general it may be desirable to allow the applied layer dry after application and before annealing. Suitable drying arrangements and times are set out above.

It may also be desirable to anneal following dip-coating. Annealing may take place after each dip-coat is applied. For example the polymer matrix may comprise a first layer only (i.e. no second layer) and that layer is optionally annealed. Alternatively the polymer matrix may comprise first and second layers and the first layer and the second layer are desirably each annealed. If dip-coating is applied then it is desirable to anneal following dip-coating and desirably after each dip-coating applied.

Annealing may take place at the temperatures set out above. In general it may be desirable to allow the applied layer dry after application and before annealing. Suitable drying arrangements and times are set out above. Suitable annealing parameters may be at a temperature in the range from about 100 to about 300 °C such as, from about 120 to 290 °C, for example from about 150 to about 260 °C, such as from about 180 to about 250 °C, such as about from about 180 to about 230 °C, suitably from about 200 to about 220 °C, particularly from about 205 to about 220 °C, for example 208 to about 217 °C such as about 210-215 °C.

Desirably the electrode of the invention with any of the constructions set out above has a polymer formed from phenylene diamine (PD) such as *o*-PD, *m*-PD and *p-*PD applied thereto. In particular it is desirable that the polymer matrix includes a polymer made from *o*-PD. The PD may be applied by electro-polymerisation to form the desired polymeric material. Application of PD and in particular *o*-PD increases permselectivity.

Typical permaselective barriers are 1 - 500 µm thick, such as from about 2 to 400µm thick, for example 3 to 200 µm thick, more desirably 10-100 µm thick. It is desirable that the polymer matrix of the invention is of such a thickness.

The conducting substrate can be constructed on any surface that can be charged with an electric voltage. Substrates of the present invention will be self-supporting, that is they do not rely on any other material for support (for example to hold them together). Their structural integrity is sufficient. The conducting substrate may comprise a non-metallic conductor such as carbon fibres or metallic conductors including those constructed of noble metals such as Pt, Ag, Au, Ru, Rh, Pd, Re, Os, Ir and combinations thereof. It will be appreciated that the conducting substrate may be formed by a conducting material coated onto a support such as a support comprising non-conductive material. The conducting substrate may take the form of a length of conducting material such as a wire *e*.*g*. Pt or carbon fibre.

For applications within the body it is desired that the electrode be minimally invasive. For that reason it is desirable that the electrode has dimensions no greater than 100 mm in length, 0.5 mm in cross-sectional width and no less than 10 mm in length. Typical dimensions range from 10 mm to 100 mm.

Suitable materials for forming the layer include halogenated materials such as fluorinated or chlorinated materials in particular, perfluoro, perchloro and perfluorochloro polymers. In particular of interest are ionomers such as a sulfonated tetrafluorethylene copolymer. One suitable commercially available material is Nafion^{®} by DuPont. Nafion® may be considered a tetrafluoroethylene-perfluoro-3,6-dioxa-4-methyl-7-octenesulfonic acid copolymer (see for example CAS 31175-20-9).

Sensors which are based on electrodes of the invention have been fully developed and characterised to determine sensitivity, selectivity and stability. The biosensors have been successfully used in the target "*in-vivo* " brain environment to monitor levels of target species.

The invention also extends to a method of constructing an electrode as described above. Such an electrode may additionally be provided with the additional features/be constructed as set out above.

In particular the present invention provides a method of forming an electrode comprising:
(i) providing a conducting substrate;
(ii) forming a polymer matrix on the substrate the polymer matrix comprising:
   a) a first layer, or
   b) first and second layers wherein the second layer is applied to the first,
   the polymer matrix forming a permselective barrier for the conducting substrate; the, or each layer comprising a first pre-coat or first and second pre-coats said first pre-coat being formed by:
   (a) depositing a liquid form of at least one halogenated polymer such as fluorinated or chlorinated polymers onto a substrate, and
   (b) allowing the material to dry; and
      said first and second pre-coats being formed by:
   (c) depositing a liquid form of at least one halogenated polymer such as fluorinated or chlorinated polymers onto a substrate,
   (d) allowing the material to dry;
   (e) depositing a liquid form of at least one halogenated polymer such as fluorinated or chlorinated polymers, onto the dried material, and
   (f) allowing the material to dry;
   said first layer being adhered to the conducting substrate and said second layer being adhered to the first layer by depositing a liquid form of at least one halogenated polymer such as fluorinated or chlorinated polymers on the layer; and utilising the material deposited on the layer to adhere the layer to the conducting substrate or the first layer so as to form the permselective barrier for the conducting substrate.

The invention also provides an electrode bundle comprising at least two implantable electrodes, one electrode for selectively detecting in use a first species, and the second electrode for selectively detecting, in use, a second species each electrode having applied thereto the same polymer matrix which forms a permselective matrix and the electrodes arranged for application of different potentials. An electrode bundle comprising at least two implantable electrodes, one electrode for selectively detecting a first species, and the second electrode for generating the first species or for selectively detecting a second species, each electrode having applied thereto the same polymer matrix which forms a permselective matrix and the electrodes arranged for application of different potentials. This provides a very effective mechanism of creating an electrode bundle which can be implanted as a single implant. For example, it is possible to coat all of the electrodes at a given time with the same coating yet have them selectively detect different target species.

Each electrode within the bundle may be an electrode as defined above. This means that not only will the electrode bundle be selective it will also be biocompatible. Desirably the electrodes are each configured for detecting a given species selected from NO, CO, H₂ and O₂. This means that an electrode bundle of the invention has the ability to detect two or more gaseous species within the body. These species can be detected in real-time and changes in concentration can thus be monitored in real-time. Utilising differences in applied potential may eliminate any requirement for a reconfiguration of the membrane formed. The potential can be a negative value (for example -550 mV) to measure a first species such as O₂ through a reduction reaction. There will then be no interference from other species such as NO which are measured through oxidation potential (for example +900 mV).

In one arrangement the electrode bundle comprises a H₂ generation electrode for generating the H₂ in-situ. It is desirable that in addition to having an electrode which is capable of detecting the species that there is also a H₂ generation electrode for generating the H₂ in-situ. Such an electrode bundle also desirably includes a H₂ detection electrode. This means that blood-flow can be measured in real-time and fluctuations in concentration can be adjusted for any deviations in blood flow rates. Again this leads to more accurate measurement and to real-time monitoring of target species. It is apparent that the present invention with the monitoring of blood flow such as by determination of the depletion rate of H₂ allows for the measurements to take into account blood flow. In particular accurate measurement of any given species could be confounded by changes in blood flow. The present invention allows for long-term real-time blood flow monitor which can be correlated against the species detected so that accurate measurement of species can be carried out which in turn may allow accurate calculation of other parameters such as metabolic rates.

It may also be desirable to include within an electrode bundle of the invention an oxidase enzyme-based electrode. This means about a target species which are not normally electroactive can be detected by conversion to an electroactive species. For example the oxidase enzyme-based electrode may incorporates glucose oxidase (GOx), lactate oxidase (LOx) or glutamate oxidase (GluOx). Indeed the electrode bundle of the invention may include combinations of same. Monitoring the levels of H₂O₂ produced by enzymatic action allows observation of minute changes in the target molecule concentrations at a real-time level. Thus, incorporation of glucose oxidase (GOx), lactate oxidase (LOx) or glutamate oxidase (GluOx) into the biosensor allows measurement of real-time fluctuations in glucose, lactate, or glutamate concentrations respectively. Real-time *in-vivo* neurochemical monitoring of these molecules has huge potential in determining and finding possible cures for a number of neuro-degenerative and psychiatric disorders.

Other species can also be detected within such an electrode bundle by provision of a suitable sensor. One further example such as oxidase enzyme-based biosensors, which in the presence of O₂ liberate electroactive H₂O₂. Oxidase-based biosensors work by electrochemically detecting H₂O₂ produced through the enzyme-catalysed oxidation reaction with their corresponding target substrate. The main basis for the GOx dominance in this field is because of the importance of glucose monitoring. For example glucose monitoring is important in relation to the disease diabetes mellitus. Glucose determination in various body fluids, such as blood, plasma and urine. Suitable sensors as described and claimed in co-pending Irish Patent Application No. S2007/0774 filed on 24 October 2007 and assigned to the present Applicant are suitable for use as part of or independently to (for example in conjunction with) an electrode bundle of the present invention.

It is apparent therefore that the present invention allows for two or more species can be detected simultaneously and without interference. The electrode bundle will generally be in an implantable form for implantation into an animal, more specifically a human body. In one arrangement the electrode bundle is for insertion into the body for monitoring of species in a body fluid such as ECF.

The invention extends to an electrode and a method substantially as described herein with reference to the examples.

### Brief Description of the Drawings

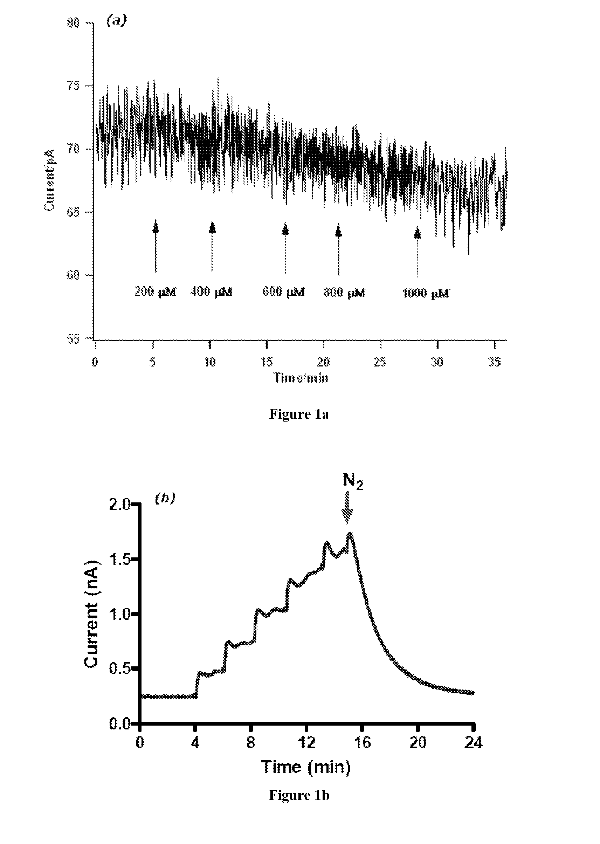

**Figure 1****: *(a)*** Typical *in vitro* response of an NO sensor to 200 nM NO injections, followed by verification of the NO signal by bubbling N₂ into the electrochemical cell containing the final concentration of 1 mM NO; ***(b)*** Typical *in vitro* response of an NO sensor to 200 µM ascorbic acid (principal endogenous interferent) injections.

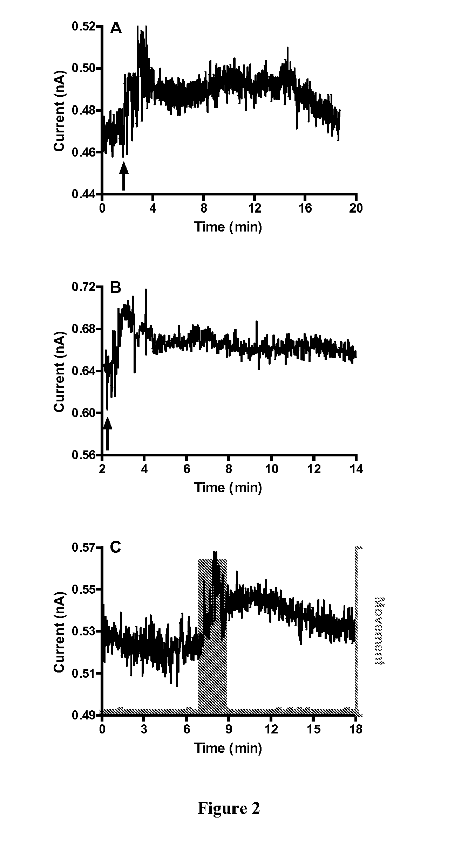

**Figure 2****:** Preliminary *in vivo* data obtained from an NO sensor (Type 1) implanted in the striatum of a freely-moving male Wistar rat (*ca*. 250 g): (A) Response to a subcutaneous (s.c.) NO injection (0.3 mM, 0.5 mL); (B) Response to intraperitoneal (i.p.) injection of L-arginine (300 mg/kg, 2 mL); (C) Current changes in response to movement (gray area) of the animal. *In vitro* calibration data suggests concentration changes of between 5-10 nM NO.

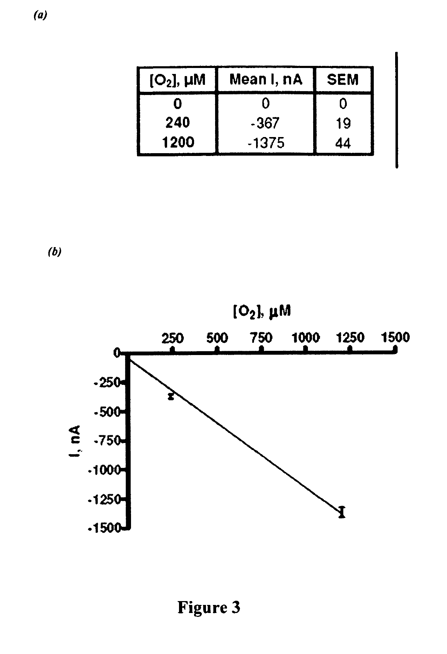

**Figure 3*****(a)*** is a Table of results for oxygen calibrations in PBS (pH 7.4) buffer solution at 21°C using bare Pt electrodes (n=18). **Figure 3(b)** shows the current-concentration profile for oxygen calibrations in PBS (pH 7.4) buffer solution at 21°C using bare Pt electrodes (n=18). Background values were subtracted. Mean background current = -408 nA. CPA carried out a -650 mV *vs*. SCE.

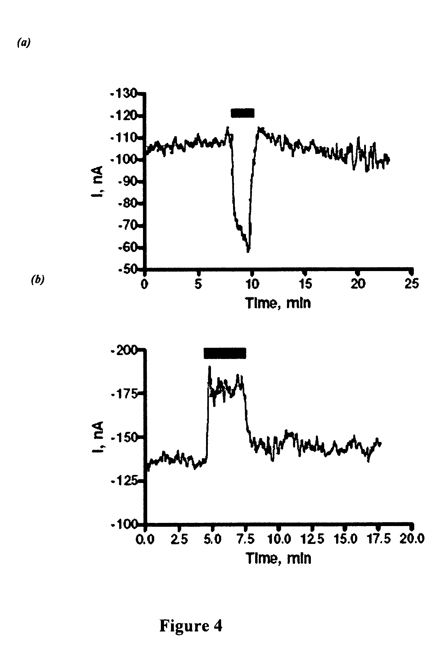

**Figure 4(a)** shows a typical example of a 3-minute period of hypoxia monitored by a pre-coat modified (Type 1) Pt electrode in the striatum of a freely-moving rat. The horizontal bar above the trace indicates the period of administration of the N₂/air mixture. **Figure 4(b)** shows a typical example of a 3-minute period of hyperoxia monitored by a pre-coat-modified (Type 1) Pt electrode in the striatum of a freely-moving rat. The horizontal bar above the trace indicates the period of administration of the O₂/air mixture.

### Detailed Description of the Drawings

The present inventors have addressed the problems associated with the present invention using a novel micro-electrochemical sensor. The latter is a device which allows one to monitor changes in concentrations of particular target species in the brain extracellular fluid in real time. The design of the sensor must ensure appropriate sensitivity and selectivity for the target species. Such devices generally consist of 5 cm length of an electrode substrate such as a Teflon-coated Platinum wire; one end is soldered to a gold clip which provides rigidity facilitating electrical contact to the instrumentation. The other end contains the active surface which is the site where the electrochemical redox reaction takes place.

The present inventors have modified this active surface with various polymers and enzymes (biosensors) to maximise two of the most important parameters - sensitivity and selectivity. By applying a suitable potential profile, the electrochemical sensors can record changes in the concentrations of a variety of substances in the extracellular fluid with sub-second time resolution over extended periods.

The present invention provides an *in vivo* sensor for brain ECF levels of NO. The sensor was formed by combining the Nafion® Pre-coat method with electropolymerised *o*-PD as electrode modifying layers. Excellent permselectivity was achieved by using a combination of Nafion® and *o*-PD protective polymers. A comprehensive study of Nafion® application to Pt electrodes was carried out in order to obtain maximum selectivity against biological interferents using AA as a model interferent (see **Figure 1(a)****)**. The present inventors obtained no increase in current upon increasing concentrations of AA and DA with a 286:1 selectivity ratio for NO₂⁻. Selectivity against NO₂⁻ is more than sufficient for *in vivo* applications.

Apart from being highly selective against interferents, *in vivo* sensors must be highly sensitive towards the target analyte. Nafion® Pre-coat electrodes proved to be very sensitive to NO displaying a current density of 13.04 µA/cm²/µM (see *Figure 1(b)*, Appendix 1). The current density obtained for Pt electrodes modified with the Nafion® Pre-coat method was more favourable than the current density obtained with unmodified bare Pt electrodes (8.96 µA/cm²/µM).

The limit of detection ("LOD") is also an important electrode characteristic for *in vivo* NO sensors. The LOD is generally taken as the concentration at which one is 95% confident the analyte is present in the sample. The LOD is affected by the precision of measurements and by the magnitude of the blanks (sample without analyte). The LOD is calculated by 3 x S.D. of the blank/M where M represents the sensitivity of the electrode. An LOD of 234 nM was achieved for our Pt electrodes modified with (5 pre-coats) 2 coats of Nafion® annealed at 210 °C after each coat. The detection limit achieved for our NO sensor appears to be greater than the detection limits obtained by various research groups, including Malinski and Taha (10 nM), Friedemann *et al*. (35 nM), and WPI (300 pM for their ISONOPMC sensor). Although these detection limits are relatively low, these specific NO sensors have issues when *in vivo* as discussed above.

Further electrode characterisation of the sensor of the present invention was carried out by examining the influence of physiological concentrations of O₂ and temperature effects on NO sensitivity. The sensitivity of the sensor was not affected by physiological concentrations of O₂ or by an increased temperature of 37 °C. One of the major problems encountered in attempting to design the *in vivo* sensor for brain ECF concentrations of NO was response time. Calibrating the electrode between a concentration range of 0 to 1 µM NO at 25 °C displayed a response time of 33.67 ± 3.71 s. The response time was improved however when operating the sensor at an increased temperature of 37 °C (14.00 ± 2.52 s).

By combining *o*-PD with a given number of Nafion® Pre-coat applications the necessary selectivity was maintained but the response time decreased.

Two NO sensors were constructed by combining the novel Pre-coat method with *o*-PD. Improved response times (*ca*. 10 s) were achieved for both NO sensors compared with the response times achieved for our original NO sensor (*ca*. 30 s) constructed solely from Nafion^{®} when calibrating at room temperature. Although sensors modified with both *o*-PD and a reduced number of Nafion® Pre-coats displayed a reduction in AA and NO₂ - selectivity when compared with selectivity obtained for electrodes modified with (5 pre-coats) 2 coats of Nafion® annealed at 210 °C after each coat (see the Lowry Analyst paper - referenced above), the permselectivity of NO sensors of the invention against AA and NO₂⁻ is acceptable for an operational *in vivo* NO sensor.

Two other NO sensors, Pt electrodes modified with (1 pre-coat) 2 coats of Nafion®, annealed after each coat at 210 °C followed by polymerisation with *o*-PD for 30 minutes (Type 2), and Pt electrodes modified with (2 pre-coat) 1 coat of Nafion®, annealed at 210 °C followed by 1 Nafion® application from 5% commercial solution annealed at 210 °C and finally polymerisation with *o*-PD for 30 minutes (Type 3) displayed detection limits of 557 nM and 240 nM respectively. The *o*-PD polymer layer has the added benefit of improving biocompatibility. We have also found that if we increase the annealing temperature (> 210 °C) the Nafion® layer tends to be less structurally compromised (i.e. exhibits less surface cracking) than layers formed at lower temperatures.

The development of sensitive, selective and fast NO *in vitro* sensors lead to the possibility of examining the effects of lipids and proteins on electrode sensitivity. Protein and lipid adsorption did not deter the working capacity of our NO sensors. The positive results regarding NO sensitivity (improved for Types 2 & 3), selectivity, response time (improved for Types 2 & 3), O₂ effects on NO sensitivity, temperature effects on NO sensitivity (no temperature effects) and effects of lipid and protein adsorption indicate the ideal suitability of these three NO electrodes for detecting NO *in vivo.* A complete *in vivo* characterisation has been carried out for the Type 1 sensor and it is clear that Type 2 and Type 3 will exhibit similarly desirable characteristics and with a faster response time. Characteristics confirmed include (i) high sensitivity to substrate; (ii) freedom from fouling by endogenous macromolecules (lipids and proteins); (iii) insignificant interference from reducing agents, especially ascorbic acid, present in the tissue, and (iv) insensitivity to changes in oxygen partial pressure and temperature under normal physiological conditions. **Figure 2** shows *in-vivo* data for Type 1 electrodes. Comparative data can be obtained with Type 2 and Type 3 (see below) electrodes but with faster response times.

*In vivo* NO sensors must have a rapid response, high sensitivity for NO and high selectivity against interferents. Three electrodes that may have the necessary attributes are:
***Type 1:*** Pt electrodes modified with (5 pre-coats) 2 coats of Nafion^{®} annealed at 210 °C after each application.
***Type* 2:** Pt electrodes modified with (1 pre-coat) 2 coats of Nafion^{®} annealed at 210 °C after each application followed by polymerisation with *o*-PD.
***Type* 3:** Pt electrodes modified with (2 pre-coats) 1 coat of Nafion^{®} annealed at 210 °C followed by dip-coating with a 5% solution of Nafion® followed by annealing at 210 °C and then polymerisation with *o*-PD.

### Electrode Preparation

This project employed the use of Pt working electrodes. Pt disk electrodes were prepared from Teflon^{®}-coated platinum/iridium (Pt/Ir 90%/10%) wire (125 µm bare diameter, 160 µm coated diameter (5T), Advent Research Materials, Suffolk, UK). The length of the Pt electrodes were approximately 5 cm. 5 mm of the Teflon^{®} insulation was carefully cut from one end of the 5T Pt wire. A gold plated clip was attached to this end of the Pt wire to provide rigidity and electrical contact. The other end of the wire was cut with a sharp scalpel to expose a Pt disk, which served as the active surface.

The working electrodes used throughout this project where either unmodified Pt disk electrodes or Pt disk electrodes modified by Nafion^{®}, or *o*-PD.

### Application of Nafion^{®}

Nafion^{®} was applied to Pt electrodes by a variety of different methods in order to establish the best method of Nafion^{®} application for selectivity against electroactive interferents. The various methods of Nafion^{®} application used throughout this work are explained in the following sections:

### (1) Nafion^{®} cast at room temperature

This involved dipping the active surface of the electrode into 5% Nafion^{®} solution. A Nafion^{®} droplet (approximately 5 µl) is placed onto a watch glass using a syringe. Almost immediately after the Nafion^{®} droplet has been placed onto the watch glass the electrode is placed into the drop of Nafion^{®} and then immediately taken out (1 to 2 seconds). The Nafion^{®} coat is then let air dry for 2 minutes between successive applications.

### (2) The Nafion^{®} Pre-coat Application

The pre-coat method involves placing a fixed volume (5 µl) of Nafion^{®} onto a watch glass using a syringe. The Nafion^{®} droplet is allowed to air dry at room temperature for 5 minutes. After the solvent (5% Nafion^{®} dissolved in aliphatic alcohols) from the initial droplet has evaporated, further individual droplets are placed on top of the original droplet using the same procedures previously outlined for the initial drop. What results from solvent evaporation is a concentrated layer of Nafion^{®} on the watch glass.

After 1-5 drops (called 1-5 pre-coats) have been placed onto the watch glass, a final drop of Nafion^{®} is placed onto the concentrated pre-coat of Nafion. The active surface of the electrode is then dipped into the Nafion^{®} concentrated layer. The electrode is then removed immediately from the concentrated layer of Nafion^{®} and let air dry at room temperature for 2 minutes. The purpose of the fresh Nafion^{®} droplet is to adhere the concentrated Nafion^{®} layer to the electrode. The electrode is then placed into an oven and annealed for 5 minutes at 210 °C. After the annealing process has been completed, the electrode can be coated again by using the same procedure i.e. placing another fresh drop of Nafion^{®} onto the concentrated Nafion^{®} layer (situated on the watch glass) and then dipping the electrode into the concentrated layer to obtain a further Nafion^{®} pre-coat. This modified electrode is air dried for 2 minutes and then annealed at 210 °C for 5 minutes. This pre-coat fabrication can be carried out numerous times.

### Application of o-PD

The various types of working electrodes that were modified with *o*-PD are described in detail in the following sections:

### (1) o-PD

*o*-PD was electroploymerised onto Pt disk electrodes by applying a constant potential of +700 mV *vs*. SCE(Saturated calomel electrode). 1 or 2 Pt electrodes were electroploymerised at a time in 300 mM monomer solution made up in deoxygenated H₂O. During polymerisation the electrochemical cell was kept under an N₂ atmosphere as the monomer is readily oxidisable in air. Electrodes were polymerised for 30 minutes. After 30 minutes of polymerisation with *o*-PD, a visible (black colour) selective membrane was formed over the active surface of the electrode. Fresh monomer solution was made up for each new set of polymerisations.

### (2) Application of (1 pre-coat) 2 coats of Nafion^{®}, annealed after each coat at 210 °C followed by polymerisation with o-PD for 30 minutes (Type 2)

2 applications of 1 Nafion^{®} pre-coat (explained in (2) of Nafion^{®} application above) were annealed at 210 °C after each application. Nafion^{®} application was followed by *o*-PD polymerisation for 30 minutes. Nafion^{®} applications were annealed at 210 °C before polmerisation with *o*-PD.

### (3) Application of (2 pre-coat) 1 coat of Nafion^{®}, annealed at 210°C followed by 1 Nafion^{®} application from 5% commercial solution annealed at 210 °C and finally polymerisation with o-PD for 30 minutes (Type 3)

1 application of 2 pre-coats of Nafion^{®}, annealed at 210 °C (described in (4) of Nafion^{®} application above), followed by 1 Nafion^{®} application by dipping into the 5% commercial solution (described in (1) of Nafion^{®} application above) with an annealing temperature of 210°C and finally polymerised with *o*-PD for 30 minutes.

### Electrochemical Experiments

### Cell design

Experiments were carried out in an electrochemical cell at room temperature (25 °C) or 37 °C. Electrochemical cells were constructed in house. A standard 3 electrode set up was used which consisted of an auxiliary, reference and working electrode.

The top of the cell was constructed from Teflon^{®} and consisted of an inlet for purging with N₂, air or O₂. The cell top also consisted of an injection port with a removable lid. The cell itself was a glass container (25 ml) with a flat base (convenient for using a magnetic stirrer).

### Constant Potential Amperometry (CPA)

CPA was used to calibrate Pt electrodes (bare Pt and unmodified Pt) for AA, UA, 5-HT, DOPAC, 5-HIAA, DA, glutathione, HVA, NO₂⁻, H₂O₂, O₂ and NO. AA, UA, 5-HT, DOPAC, 5-HIAA, DA, glutathione, HVA, NO₂⁻, H₂O₂ and NO calibrations were performed at either +700 or +900 mV vs. SCE. O₂ calibrations were performed using a CPA of -550 mV vs. SCE. All CPA experiments were recorded using a Gateway GP6-350 computer with data acquisition performed using the commercial PowerLab/400 program: Chart for windows V3.4 (ADInstruments Ltd.). A low-noise potentiostat (EMS) was used in all experiments.

### Experimental Details

15/20 ml PBS was purged with N₂ for approximately 30 minutes. Pt electrodes were let settle in PBS, under the influence of an applied potential until a stable background current was achieved. Stable background currents were achieved after 1-2 hours for most Pt electrodes with the exception of Pt electrodes modified by *o*-PD, which required overnight stabilisation.

O₂ and NO CPA experiments differ slightly from all other calibrations performed. 3 point calibrations performed for O₂ are achieved by deoxygenating the electrochemical cell using N₂ until no O₂ remains in the buffer, once no O₂ reduction signal is being detected by the working electrode the buffer is bubbled with an air pump to achieve *ca.* 200 µM O₂, and then finally bubbled with pure oxygen to achieve a saturated PBS solution with an O₂ concentration of *ca.* 1,200 µM.

The reported high reactivity of NO with O₂ and the presence of *ca.* 50 µM O₂ in the ECF of the brain made it necessary to calibrate the signal response of NO sensors in PBS containing *ca.* 50 µM O₂ as well as in N₂ saturated solution. This is achieved by saturating the electrochemical cell with air (*ca*. 200 µM) and then bubbling N₂ into the cell to bring the concentration of O₂ back down to *ca.* 50 µM. Only one working electrode can be calibrated for NO (polarised at +900 mV vs. SCE) at a time as the second working electrode (polarised at -550 mV vs. SCE) present in the electrochemical cell is used to monitor the concentration of O₂ in the buffer.

Calibration steps for the different species used during this study are summarised in **Table 1:**

**Table 1 Calibration steps used for various elecroactive species examined during the course of this project.**

| **Substrates:** | **Calibration steps:** |
|---|---|
| AA: | 0, 200, 400, 600, 800, 1,000 µM |
| DOPAC: | 0, 20, 40, 60, 80, 100, 120 µM |
| Glutathione: | 0, 10, 20, 30, 40, 50, 60 µM |
| DA: | 0, 0.01, 0.02, 0.03, 0.04, 0.05, 0.06, 0.07, 0.08 µM |
| HVA: | 0, 5, 10, 15, 20 µM |
| H₂O₂: | 0, 200, 400, 600, 800, 1,000 µM |
| 5-HIAA: | 0, 10, 20, 30, 40, 50, 60 µM |
| 5-HT: | 0, 1, 2, 3 µM |
| NO: | 0, 0.2, 0.4, 0.6, 0.8, 1.0 µM |
| NO₂⁻: | 0, 200, 400, 600, 800, 1,000 µM |

### Data analysis

Current-concentration plots and raw data plots, where prepared using Microsoft Excel (Office 2000). Slopes and linear coefficients were also obtained using Microsoft Excel. Student t-tests were performed using GraphPad Instat (version 3.05, 32 bit for Win 95/NT, Graphpad software Inc., USA).

### Biocompatibility

The three NO sensors display varying degrees of ability in preventing electrode poisoning. Type 1 (T1) has a permselective membrane constructed completely of annealed Nafion^{®}. The sensitivity of T1 was found to be significantly reduced by storing electrodes in 10% BSA (Bovine serum albumin, *P* = 0.0346) or 10% PEA (L-α-phosphatidylethanolamine (type II-S)) *P* = 0.0008) for 24 hrs compared with sensitivity obtained for E1 electrodes stored in air. These results indicate that these electrodes are susceptible to electrode fouling when placed in 10% BSA or 10% PEA over a 24 hr period (see Table 3). Electrode fouling does not continue to become progressively worse when electrodes are placed in a 10% BSA solution (*P* = 0.2068) or a 10% PEA solution (*P* = 0.4676) for an extra 48 hrs.

Type 2 (T2) electrodes did not show a significant decrease in NO sensitivity when placed in 10% BSA (*P* = 0.0204 indicates a significant increase in NO sensitivity) or 10% PEA for 24 hrs (*P* = 0.7478). The superior ability to prevent electrode fouling for sensors modified with *o*-PD compared with sensors modified only with annealed Nafion^{®} emphasises the greater biocompatibility of *o*-PD compared with Nafion^{®} over a 24 hr period.

NO sensitivity was not significantly reduced for T3 electrodes placed in a 10% BSA (*P* = 0.3275) or a 10% PEA (*P* = 0.8415) solution for 24 hrs. Increased selectivity against protein and lipid fouling for Pt electrodes modified with (2 pre-coats) 1 coat of Nafion^{®} annealed at 210 °C followed by polymerisation with *o*-PD compared with results obtained for Pt electrodes modified with (5 pre-coats) 2 coats of Nafion^{®} annealed at 210 °C after each application must be due to the presence of polymerised *o-*PD.

### Response Time

The fastest response time achieved with modified Pt electrodes was by those modified with (1 pre-coat) 2 coats of Nafion^{®} annealed at 210 °C after each application followed by polymerisation with *o*-PD (11.50 ± 7.50 s, n = 3, Type 2), however these electrodes did not exhibit as fast a response time as unmodified Pt electrodes (6.79 ± 1.07, n = 28). Increasing the number of Nafion^{®} pre-coats from 1 to 2 (Pt electrodes modified with (2 pre-coats) 1 coat of Nafion^{®} annealed at 210 °C followed by polymerisation with *o*-PD) increased the electrodes response time (13.40 ± 0.50 s, n = 11) - Type 3 (T3). The slowest response time displayed by any of the modified Pt electrodes tested was obtained with Pt electrodes modified with the largest number of pre-coats, (5 pre-coats) 2 coats of Nafion^{®} annealed at 210 °C after each application (33.67 ± 3.71, n = 14) - Type 1.

In general, it was observed that the greater the number of Nafion^{®} pre-coats applied to the electrode surface, the slower the electrode response (see **Table 2**). The reason for obtaining a slower electrode response with increased number of Nafion^{®} applications is because NO molecules have to diffuse through the Nafion^{®} layer before being oxidised at the electrode surface, therefore the thicker the Nafion^{®} permseletive membrane, the greater the distance the NO molecules have to diffuse before reaching the electrode.

**Table 2.**

| **Electrode** | **Response time (s)** |
|---|---|
| Bare Pt | 6.79 ± 1.07 (28) |
| T1 | 33.67 ± 3.71 (14) |
| T2 | 11.50 ± 7.50 (3) |
| T3 | 13.40 ± 0.50 (11) |

**Table 2** shows a comparison of the response times for NO at Pt disk electrodes (Bare Pt), Pt disk electrodes that had been modified with (1 pre-coat of Nafion^{®}) 2 coats annealed at 210 °C after each coat followed by polymerisation with *o-*PD for 30 minutes (T2), Pt disk electrodes that had been modified with (2 pre-coats of Nafion^{®}) 1 coat annealed at 210 °C followed by 1 Nafion^{®} application from 5% commercial solution with an annealing temperature of 210°C and finally polymerised with *o*-PD for 30 minutes (T3) and Pt disk electrodes that had been modified with (5 pre-coats of Nafion^{®}) 2 coats, annealed at 210 °C for 5 minutes after each application (T1). CPA carried out at +900 mV vs. SCE. The number of electrodes used are in parenthesis.

Although increasing the number of Nafion^{®} pre-coats appeared to have a negative effect on electrode response time, our sensitivity results displayed in Table 3 show that by increasing the number of Nafion^{®} pre-coats it is possible to obtain increased sensitivity.

Maximum sensitivity was obtained for Pt electrodes modified with the largest number of pre-coats, (5 pre-coats) 2 coats annealed at 210 °C after each application (1.67 ± 0.08 nA/µM, n = 14)). The lowest sensitivity was achieved for Pt electrodes modified with a 1 pre-coat application, (1 pre-coat) 2 coats of Nafion^{®} annealed at 210 °C after each application followed by polymerisation with *o*-PD (0.97 ± 0.21 nA/µM, n = 3, see Table 3).

The highest selectivity against AA interference was achieved with Pt electrodes modified with (5 pre-coats) 2 coats of Nafion^{®} annealed at a temperature of 210 °C and Pt electrodes modified with (1 pre-coat) 2 coats of Nafion^{®} annealed at 210 °C after each application followed by polymerisation with *o*-PD (see Table 3). Although Pt electrodes modified with (2 pre-coats) 1 coat of Nafion^{®} annealed at 210 °C followed by polymerisation with *o*-PD are less selective that the other two NO sensors constructed, this sensors selectivity against AA is not significantly different (*P* = 0.0802) from that of Pt electrodes modified with (5 pre-coats) 2 coats of Nafion^{®} annealed at 210 °C after each application.

Electrodes with a larger number of Nafion^{®} pre-coat applications exhibit greater selectivity against NO₂⁻. The electrode modified with the largest number of Nafion^{®} pre-coat applications ((5 pre-coats) 2 coats of Nafion^{®} annealed at 210 °C after each application) showed the greatest permselective characteristics against NO₂⁻ (see Table 3).

### Summary

It was decided to examine the possibility of combining *o*-PD with a lower number of Nafion^{®} Pre-coat applications. It was thought that *o*-PD would provide the necessary selectivity against large electroactive species, while Nafion^{®} would utilise its negative repulsive effects against negatively charged interferents such as NO₂⁻.

Two new NO sensors were constructed (Type 2 coated and Type 3 coated) by combining the novel pre-coat method with *o*-PD. Improved response times were achieved for both NO sensors compared with the response times achieved for our original NO sensor constructed solely from Nafion^{®} when calibrating at room temperature. Although sensors modified with both *o*-PD and a reduced number of Nafion^{®} pre-coats displayed a reduction in AA and NO₂⁻ selectivity when compared with selectivity obtained for electrodes modified with (5 pre-coats) 2 coats of Nafion^{®} annealed at 210 °C after each coat, we considered the permselectivity of these two NO sensors against AA and NO₂⁻ to be acceptable for an operational *in vivo* NO sensor.

These two new NO sensors, Pt electrodes modified with (1 pre-coat) 2 coats of Nafion^{®}, annealed after each coat at 210 °C followed by polymerisation with *o*-PD for 30 minutes, and Pt electrodes modified with (2 pre-coat) 1 coat of Nafion^{®}, annealed at 210 °C followed by 1 Nafion^{®} application from 5% commercial solution annealed at 210 °C and finally polymerisation with *o*-PD for 30 minutes displayed detection limits of 557 nM and 240 nM respectively.

The development of sensitive, selective and fast NO *in vitro* sensors lead us to the possibility of examining the effects of lipids and proteins on electrode sensitivity. Protein and lipid adsorption did not deter the working capacity of our NO sensors. The positive results obtained regarding NO sensitivity, selectivity, response time, O₂ effects on NO sensitivity, temperature effects on NO sensitivity and effects of lipid and protein adsorption indicate the strong possibility of our three NO electrodes detecting NO *in vivo*.

An overall comparison of the electrode characteristics for the three most successful NO sensors produced is given in **Table 3:**

**Table 3.**

| | | | |
|---|---|---|---|
| **Electrode** | **NO Sensitivity 25 °C** | **NO Sensitivity 37 °C** | **NO Sensitivity 50** µ**M O₂** |
| T1 | 1.67 ± 0.08 nA/µM | 1.53 ± 0.28 nA/µM | 1.34 ± 0.19 nA/µM |
| T2 | 0.97 ± 0.21 nA/µM | - | - |
| T3 | 1.38 ± 0.21 nA/µM | - | - |
| | | | |
| **NO Sensitivity PEA** **(24 hr)** | **NO Sensitivity [00108] BSA** **(24 hr)** | **NO Sensitivity [00109] PEA** **(72 hr)** | **NO Sensitivity BSA** **(72 hr)** |
| 0.97 ± 0.12 nA/µM | 1.19 ± 0.24 nA/µM | 1.122 ± 0.001 nA/µM | 0.87 ± 0.05 nA/µM |
| 0.87 ± 0.10 nA/µM | 1.87 ± 0.21 nA/µM | - | - |
| 1.45 ± 0.30 nA/µM | 1.08 ± 0.10 nA/µM | - | - |
| | | | |
| **Response time 25 °C** | **Response time 37 °C** | **[00110] AA Selectivity 25 °C** | **NO₂⁻ Selectivity 25 °C** |
| 33.67 ± 3.71 s | 14.00 ± 2.52 s | -0.11 ± 0.08 nA/mM | 0.03 ± 0.02 nA/mM |
| 11.50 ± 7.50 s | - | -0.02 ± 0.02 nA/mM | 1.90 ± 0.15 nA/mM |
| 13.40 ± 0.05 s | - | 0.07 ± 0.07 nA/mM | 0.29 ± 0.17 nA/mM |

Table 3 gives a summary of the electrode characteristics obtained for various Pt disk electrodes that had been modified with (1 pre-coat of Nafion^{®}) 2 coats annealed at 210 °C after each coat followed by polymerisation with *o*-PD for 30 minutes (T2); (2 pre-coats of Nafion^{®}) 1 coat annealed at 210 °C followed by 1 Nafion^{®} application from 5% commercial solution with an annealing temperature of 210 °C and finally polymerised with *o*-PD for 30 minutes (T3); and (5 pre-coats of Nafion^{®}) 2 coats, annealed at 210 °C for 5 minutes after each application (T1) under various conditions. CPA carried out at +900 mV. The electrodes were tested for NO sensitivity (nA/µM) at a temperature of 25 °C and 37 °C, NO sensitivity in the presence of 50 µM O₂, after being immersed in 10% BSA or 10% PEA for 24 or 72 hrs, electrode response time at 25 °C and 37 °C, and selectivity (nA/mM) against AA and NO₂⁻.

### Oxygen

Pre-coated Pt electrodes prepared using the pre-coat method (e.g. any one of Type 1-3 (such as T2 and T3)) provide protection against surface poisoning yet do not show any significant difference in the O₂ sensitivity compared with bare Pt (Bare Pt: 328 ± 15 nA, n = 4; pre-coat-modified: 231 ± 36.43 nA, n = 3, *P* = 0.1333) indicating that the membrane formed by the pre-coat method is permeable to molecular O₂, as demonstrated below.

The selectivity of pre-coat-modified Pt electrodes for O₂ relative to a variety of potential interferents present in brain ECF has been characterised *in vitro.* The compounds tested included the neurotransmitters dopamine (DA) and 5-hydroxytryptamine (5-HT), their metabolites 3,4-dihydroxyphenylacetic acid (DOPAC), homovanillic acid (HVA), and 5-hydroxyindoleacetic acid (5-HIAA), and other electroactive species such as L-tyrosine, L-cysteine, L-tryptophan, L-glutathione, dehydroascorbic acid, and the purine metabolite uric acid (UA). The results are summarised in the **Table 4** below and although in most cases there was little or no immediate response to the interferent, in some cases slight positive or negative drifts were observed over several minutes. However, it is clear from the results in Table that the above compounds have no appreciable effect on the sensor response for O₂. Thus, these *in vitro* results suggest that direct interference by endogenous compounds is minimal, and that these electrodes will have interference free signals for O₂ *in vivo* (*see* **Figure 3** Pt O₂ data).

**Table 3** *In vitro* response of pre-coat-modified (Type 1) Pt electrodes (*n* = 4) for a variety of potential interferents † expressed as a percentage of the O₂ (50 µmol/L) currents at physiologically relevant concentrations.

| **Interferents** | **O₂ (%)** |
|---|---|
| | 100 (58 nA) |
| DHAA | 1.13 |
| Glutathione | 0.48 |
| Dopamine | < 0.01 |
| DOPAC | < 0.01 |
| 5-HT | < 0.01 |
| HVA | 0.17 |
| Uric acid | < 0.01 |
| 5-HIAA | < 0.01 |
| L-Cysteine | 1.32 |
| L-Tryptophan | < 0.01 |
| L-Tyrosine | < 0.01 |

| | |
|---|---|
| DHAA, dehydroascorbic acid; DOPAC, 3,4-dihydroxyphenylacetic acid; 5-HT, 5-hydroxytryptamine; HVA, homovanillic acid; 5-HIAA, 5-hydroxyindoleacetic acid. †100 µM interferent, or brain extracellular fluid (ECF) concentration if known: Glutathione (50 µM); Dopamine (0.05 µM); DOPAC (20 µM); 5-HT (0.01 µM); HVA (10 µM); Uric acid (50 µM); 5-HIAA (10 µM); L-Cysteine (50 µM). | |

All data was recorded in PBS (pH 7.4). Constant potential amperometry (CPA) involved applying a potential of -650 mV vs. SCE to the working electrodes. Three-point calibrations were carried out by introducing various concentrations of oxygen to the cell. An [O₂] of 0 µM was achieved by deoxygenating the buffer solution with N₂ gas. An [O₂] of 240 µM was achieved by bubbling atmospheric air through the buffer solution. An [O₂] of 1200 µM was achieved by bubbling pure O₂ gas through the buffer solution. These concentrations were used to allow regression analysis to be performed. The data is reported as mean ± SEM, n = number of electrodes, unless stated otherwise. All calibration plots were linear; therefore the slope (nA/µM) is used as an index of sensitivity.

**Figure 3*****(a)*** is a Table of results for oxygen calibrations in PBS (pH 7.4) buffer solution at 21°C using bare Pt electrodes (n=18). **Figure 3(b)** shows the current-concentration profile for oxygen calibrations in PBS (pH 7.4) buffer solution at 21°C using bare Pt electrodes (n=18). Background values were subtracted. Mean background current = -408 nA. CPA carried out a -650 mV vs. SCE.

The oxygen data in Figure 3 is in vitro data from bare Pt electrodes - this gives the maximum sensitivity that could be expected from a Pt transducer as it is unmodified. The pre-coat (Type 1) -modified Pt sensor was tested in vitro and no difference in sensitivity was found as noted in Figure 1. In particular, it is to be noted that for oxygen calibrations performed in N₂ and air at both bare Pt and pre-coat modified (Type 1) Pt electrodes no significant difference was observed in the sensitivities of both electrode types at 200 µM O₂ (Bare Pt: 328 ± 15 nA, n = 4; pre-coat-modified: 231 ± 36.43 nA, n = 3, *P* = 0.1333) indicating that the pre-coat membrane is permeable to molecular O₂. The present inventors thus expect Type 2 and 3 pre-coats to give the same results and that these can thus also be used for O₂ measurements.

**Figure 4(a)** shows a typical example of a 3-minute period of hypoxia monitored by a pre-coat modified (Type 1) Pt electrode in the striatum of a freely-moving rat. The horizontal bar above the trace indicates the period of administration of the N₂/air mixture. **Figure 4(b)** shows a typical example of a 3-minute period of hyperoxia monitored by a pre-coat-modified (Type 1) Pt electrode in the striatum of a freely-moving rat. The horizontal bar above the trace indicates the period of administration of the O₂/air mixture. The data in Figure 4 is in vivo data for a Type 1 sensor and demonstrates that the senor does detect changes in O₂ in vivo. Again it si expected that expect Type 2 and 3 pre-coats to give the same results.

The words "comprises/comprising" and the words "having/including" when used herein with reference to the present invention are used to specify the presence of stated features, integers, steps or components but does not preclude the presence or addition of one or more other features, integers, steps, components or groups thereof. [00120] It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. An electrode comprising:
(i) a conducting substrate for detecting a target species selected from CO, H₂ and O₂;
(ii) a polymer matrix which forms a permselective barrier for the conducting substrate;
the polymer matrix comprising a layer formed by:
(a) depositing a liquid form of at least one halogenated polymer such as fluorinated or chlorinated polymers for example, perfluoro, perchloro and perfluorochloro polymers, onto a substrate,
(b) allowing the material to dry,
(c) depositing a liquid form of at least one halogenated polymer such as fluorinated or chlorinated polymers for example, perfluoro, perchloro and perfluorochloro polymers, onto the dried material,
(d) optionally repeating steps (b) and (c);
depositing a liquid form of at least one halogenated polymer such as fluorinated or chlorinated polymers for example, perfluoro, perchloro and perfluorochloro polymers, onto the layer thus formed; and
utilising the material deposited on the layer to adhere the layer to the conducting substrate so as to form a permselective barrier for the conducting substrate.

2. An electrode comprising:
(i) a conducting substrate;
(ii) a polymer matrix which forms a permselective barrier for the conducting substrate and formed from:
a. a first layer, or
b. first and second layers wherein the second layer is applied to the first, the polymer matrix forming a permselective barrier for the conducting substrate;
the, or each layer comprising a first pre-coat or first and second pre-coats said first pre-coat being formed by:
(a) depositing a liquid form of at least one halogenated polymer such as fluorinated or chlorinated polymers onto a substrate, and
(b) allowing the material to dry; and
said first and second pre-coats being formed by:
(c) depositing a liquid form of at least one halogenated polymer such as fluorinated or chlorinated polymers onto a substrate,
(d) allowing the material to dry;
(e) depositing a liquid form of at least one halogenated polymer such as fluorinated or chlorinated polymers, onto the dried material, and
(f) allowing the material to dry;
said first layer being adhered to the conducting substrate and said second layer being adhered to the first layer by depositing a liquid form of at least one halogenated polymer such as fluorinated or chlorinated polymers on the layer; and utilising the material deposited on the layer to adhere the layer to the conducting substrate or the first layer so as to form the permselective barrier for the conducting substrate.

3. An electrode according to any one of the preceding claims wherein a second layer is adhered to a first layer already applied to the substrate.

4. An electrode according to Claim 2 wherein the polymer matrix is formed by first and second layers each layer comprising a first pre-coat.

5. An electrode according to Claim 2 wherein the polymer matrix is formed by a first layer and said layer comprises first and second pre-coats.

6. An electrode according to any preceding claim wherein the polymer matrix formed is dip-coated once utilising a liquid form of at least one halogenated polymer such as fluorinated or chlorinated polymers.

7. An electrode according to any one of Claims 1 to 5 wherein the polymer matrix formed is dip-coated twice utilising a liquid form of at least one halogenated polymer such as fluorinated or chlorinated polymers.

8. An electrode according to any preceding claim wherein the polymer matrix has a polymer formed from phenylenediamine applied thereto.

9. An electrode according to Claim 8 wherein the phenylene-diamine is applied by electro-polymerisation.

10. A sensor configured for detecting a target species selected from NO, CO, H₂ and O₂ comprising an electrode according to Claim 2.

11. An electrode bundle comprising at least two implantable electrodes, one electrode for selectively detecting a first species, and the second electrode for generating the first species or for selectively detecting a second species, each electrode having applied thereto the same polymer matrix which forms a permselective matrix and the electrodes arranged for application of different potentials.

12. An electrode bundle according to Claim 11 wherein each electrode is an electrode as defined in any one of Claims 2 to 9.

13. An electrode bundle according to Claim 11 or Claim 12 wherein the electrodes are each configured for detecting a given species selected from NO, CO, H₂ and O₂.

14. An electrode bundle according to any one of Claims 11 to 13 comprising a H₂ generation electrode for generating H₂ in-situ.

15. An electrode bundle according to any one of Claims 11 to 14 further comprising an oxidase enzyme-based electrode.

16. An electrode bundle according to Claim 15 wherein the oxidase enzyme-based electrode incorporates glucose oxidase (GOx), lactate oxidase (LOx) or glutamate oxidase (GluOx).

17. A method of forming an electrode comprising:
(i) providing a conducting substrate;
(ii) forming a polymer matrix on the substrate the polymer matrix comprising:
a. a first layer, or
b. first and second layers wherein the second layer is applied to the first, the polymer matrix forming a permselective barrier for the conducting substrate; the, or each layer comprising a first pre-coat or first and second pre-coats said first pre-coat being formed by:
(a) depositing a liquid form of at least one halogenated polymer such as fluorinated or chlorinated polymers onto a substrate, and
(b) allowing the material to dry; and
said first and second pre-coats being formed by:
(c) depositing a liquid form of at least one halogenated polymer such as fluorinated or chlorinated polymers onto a substrate,
(d) allowing the material to dry;
(e) depositing a liquid form of at least one halogenated polymer such as fluorinated or chlorinated polymers, onto the dried material, and
(f) allowing the material to dry;
said first layer being adhered to the conducting substrate and said second layer being adhered to the first layer by depositing a liquid form of at least one halogenated polymer such as fluorinated or chlorinated polymers on the layer; and utilising the material deposited on the layer to adhere the layer to the conducting substrate or the first layer so as to form the permselective barrier for the conducting substrate.
